# EUROPEAN PATENT APPLICATION

(11) **EP 2 669 383 A1**
(43) Date of publication of application: **04.12.2013**
(21) Application number: 12170553.7
(22) Date of filing: 01.06.2012
(51) Int. Cl.: C12Q 1/68, C12N 15/11, G01N 33/68

(54) **New method for the isolation of the proteins bound to any kind of interesting nucleic acid sequence**

(71) Applicant: CNRS, 75016 Paris (FR)
(72) Inventor: Fuchs, Robert, 13009 Marseille (FR); Fujii, Shingo, 13010 Marseille (FR); Isogawa, Asako, 13010 Marseille (FR)
(74) Representative: Santarelli

(57) **Abstract**

The invention is to supply a novel method for isolation of the proteins bound to any kind of interesting nucleic acid sequence (Chromatin of Interest (Col)), wherein
- in a first step a Triplex Forming Tags cassette (TFT cassette) is introduced in said nucleic acid sequence of a living cell and said living cells are grown;
- in a second step cells obtained in step 1 are collected and mixed with at least one molecular probe (the TFO probe) specific of at least one of the introduced TFT sequences of the TFT cassette in conditions that permit the formation of nucleic acid triplex;
- in a third step the nucleic acid triplex formed in second step are isolated and bound proteins are analyzed,

characterized in that the TFT cassette comprises at least 2 contiguous TFT sequences.

## Description

Eukaryotic DNA is bound and interpreted by numerous protein complexes in the context of chromatin.

A description of the full set of proteins that regulate specific loci is critical to understanding gene expression and regulation.

Cellular metabolisms, such as genome maintenance, gene expression, programmed cell proliferation, and so on, are archived (indexed) in chromatin by means of specific combinations of tightly regulated proteins that carry post translational modifications and are part of protein networks.

Despite substantial characterization efforts, chromosomes remain poorly characterized cellular organelles (Kornberg and Lorch, 2007).

Understanding the way transcriptional history is encoded and stored in chromatin is a huge challenge that is presently limited by the lack of convenient procedures to isolate specific fragments of chromatin, thereafter called Chromatin of Interest (Col) and to analyze their protein content as a function of growth conditions, cell cycle, cell type,...

The term "Chromatin-of-Interest" fragment (Col fragment) refers to the complex between a specific Deoxyribonucleic acid (DNA) sequence (for ex: a given promoter region) and all of its associated proteins. The Col can be a genuine chromosomal fragment or a nucleoprotein complex assembled on an episome.

According to the invention, Chromatin of Interest (Col) is used to name the nucleotide sequence that will be isolated with the method according to the invention. Using this expression does not prejudge the fact that the sequence was known beforehand or not.

Isolation of such specific nucleoprotein fragments is a particularly demanding task given the huge size of genomes such as the human genome (3 x 10⁹ bp).

To isolate a chromatin fragment associated to a single human gene (hypothetical average size of 3 kb), one will need to isolate 1 out of 10⁶ fragments.

There is a need for methods that permit proper isolation of Col fragments.

During the past 25 years, various chromatin isolation strategies have been pursued to establish locus-specific protein composition.

While each achieved enrichment of the targeted region, none gave material in sufficient amount and purity to allow identification of bound factors.

Déjardin and Kingston (Cell 136, 175-186, January 9, 2009) described a method based on Watson-Crick hybridization to isolate telomere containing chromatin fragments.

But this technique, can only be used for telomere fragments that contain single-stranded regions (3'-overhang) able to hybridize with complementary oligonucleotide probe by Watson-Crick base pairing.

Telomeres are an exception because (1) they naturally contain single strand DNA of known sequences that can thus form Watson-Crick base pairs with a complementary probe, (2) they are over-represented because there are 92 telomeres / cell.

The described technique is thus not universal and does not permit the isolation of nucleoprotein fragment outside the end of chromosomes, that is anywhere in the chromosome.

In EP2407552 A1, published on January 18, 2012 the Applicant described a method for the isolation and identification of proteins bound to any kind of interesting nucleic acid sequence, based upon the use of a specific nucleic acid sequence tag, advantageously a specific double-stranded DNA able to form a triple helix, referred to as the Triplex-Forming Tag sequence, (TFT sequence), that will be located nearby the interesting nucleic acid sequence wherein the TFT sequence is under the form of a single double strand Poly-Purine (poly-Pu)/poly-Pyrimidine (poly-Py) sequence (or of course a poly-Py/poly-Pu sequence). In the present text the use of Poly-Purine (poly-Pu)/poly-Pyrimidine (poly-Py) sequence has to be understood as Poly-Purine (poly-Pu)/poly-Pyrimidine (poly-Py) sequence or of course a poly-Py/poly-Pu sequence.

Said TFT sequence can form a stable complex in the form of a triple helix with a specific oligonucleotide probe referred to as the Triplex Forming Oligonucleotide (TFO).

The present invention is based on the idea that multiple repeats of TFT sequences will increase the chance a single TFT sequence is available for efficient triple helix formation. This invention is to supply an optimized structure of a specific double-stranded DNA sequence tag (referred to as the Triplex-Forming Tag Cassette: TFT cassette) and of the corresponding Triplex Forming Oligonucleotides (TFO probes) to be used within the frame of the so called Chromatin-of-Interest Fragment Isolation (CoIFI) technology.

The goal of the present invention is to improve the recovery yield of the chromatin fragment of interest and to improve the signal over noise ratio. The present invention entails two major improvements: i) implementation of a Triplex-Forming Tag cassette (TFT cassette) containing multiple TFT sequences; ii) implementation of a sequential capture protocol.

The Applicant has now shown that the capture specificity of DNA fragments can be improved when using a TFT cassette defined as a double strand nucleic acid fragment comprising at least 2 TFT sequences. This improvement is particularly relevant for the capture of chromatinized DNA fragments (nucleosomes) as it increases the probability of accessibility of a TFT sequence. The possibility of a given TFT sequence to form a triplex with the complementary TFO probe is likely to depend upon the positioning of the nucleosomes. It is likely that triplex formation is favored in the linker regions between nucleosomes. Thus, using TFT cassettes will increase the chance that one of them becomes available to triplex formation.

More the Applicant has shown that the improvement is better when said TFT cassette comprises more than 2 TFT sequences, that is any number of TFT sequences over 2.

Thus the invention is to supply an improvement of the method described in EP2407552 A1 for the isolation and identification of proteins bound to any kind of interesting nucleic acid sequence (Chromatin of Interest (Col)) advantageously to any kind of interesting DNA sequence, particularly in the context of chromosomal DNA or RNA or episomal DNA in living cells or in test tubes.

In the context of the present invention, living cells include any organism that contains nucleic acid material as for example viruses, bacteria, cells, the bound protein of which have to be analyzed. The method according to the invention can be applied to prokaryotic or eukaryotic cells.

Thus, a first object of the invention relates to a new method for the isolation of the proteins bound to any kind of interesting nucleic acid sequence (Chromatin of Interest (Col)), wherein
- in a first step a Triplex Forming Tags cassette (TFT cassette) is introduced in said nucleic acid sequence of a living cell and said living cells are grown;
- in a second step cells obtained in step 1 are collected and mixed with at least one molecular probe (the TFO probe) specific of at least one of the introduced TFT sequences of the TFT cassette in conditions that permit the formation of nucleic acid triplex;
- in a third step the nucleic acid triplex formed in second step are isolated and bound proteins are analyzed,
**characterized in that** the TFT cassette comprises at least 2 TFT sequences.

According to the invention, said TFT cassette must be understood as possibly made of at least 2 TFT sequences as defined above, advantageously between 2 and 12 preferably between 3 and 10, more preferably between 5 and 7 TFT sequences.

According to the invention, in the TFT cassette each TFT sequences can be under the form of a single double strand Poly-Purine (poly-Pu)/poly-Pyrimidine (poly-Py) sequence as defined above. In the TFT cassette the TFT sequences can be all identical or all different or a mixture of identical and different TFT sequences. According to the invention the TFT sequences in the TFT cassette can be arranged in any order.

Said TFT sequences simultaneously or independently, can have a length comprised between 10 to 50 base pairs, preferably between 15 to 35 base pairs very preferably of about 20 base pairs long and can be arranged head to head or head to tail.

In the TFT cassette said TFT sequences can be contiguous (directly linked one to the other) or separated by a spacer made of 1 to 20, preferably between 1 to 10 nucleotide base pairs.

In a preferred embodiment of the invention, the TFT sequences contained in the TFT cassette can be sequences that are usually not present in the cell nucleic acid sequence in which the Col is present.

According to the first step of the invention, the TFT cassette can be in the form of a double strand linear or circular nucleic acid that is containing only the TFT cassette or can be part of double strand linear or circular nucleic acid that is containing the TFT cassette and other sequences (without contrary indication, in the present text, the use of "TFT cassette" refers to the 2 described possible forms).

In one first embodiment of the invention, the TFT cassette can be maintained in the cell as an episome. According to this embodiment the TFT cassette will be a circular nucleic acid, preferably a plasmid including in addition to the TFT cassette a viral origin.

In a second embodiment of the invention, the TFT cassette can be introduced in said nucleic acid sequence of a living cell randomly or in a directed manner that is introduced close to a predetermined Col.

When the TFT cassette insertion is made at random it will be possible to analyze unknown Col. For this the TFT cassette can be any type of DNA, advantageously further comprising a reporter sequence used as a marker for the detection of cells having said integrated DNA.

When the TFT cassette insertion is made in a directed manner via either homologous or site-specific recombination, it will be possible to analyze a known Col and the protein bound on it. The insertion of the TFT cassette will be in an area of known nucleic acid sequence of interest whose flanking sequences are known that allows introducing specifically the TFT cassette nearby the known Col to be analyzed. In this embodiment the TFT cassette can include in addition to the TFT cassette itself known flanking sequences that will permit homologous recombination between the TFT cassette and the cell nucleic acid of interest. Advantageously the TFT cassette can be a plasmid and more advantageously said plasmid can further comprise a sequence used as a marker for the detection of cells having integrated TFT cassette.

According to the first step of the invention, the TFT cassette can be introduced in said living cell through any known methods such as the calcium phosphate method (Graham, F. L. and Van Der Eb., A. J., 1973, Virology 52:456-467), the DEAE dextran method (Farber, F.,et. al.,1975, Biochem. Biophys. Acta., 390:298-311; Pagano, J. S., 1970, Prog. Med. Virol. 12:1-48), the polyornithine method (Farber, F., et.al.,1975, Biochem. Biophys. Acta., 390:298-311), the DNA microinjection method (Cappechi, M. R., 1980, Cell. 22:479-488), the polyethylene glycol (PEG)/dimethylsulfoxide(DMSO) method (Jonak, Z. L., et.al., 1984, Hybridoma 3:107-118), the trypsin/EDTA/ glycerol (Chu, G. J. and Sharp, P. A., 1981, Gene 13:197-202), the osmotic shock method (Okada, G. Y., and Rechsteiner, M., 1982, Cell 29: 33-41), a liposome fusion method (Poste, G., et. al., 1976, Methods. Cell. Biol., 14:33-71; Fraley, R. et.al., 1980, J. Biol. Chem. 255; 10431-10435; Wong, T. K., et.al., 1980, Gene 10;87-94), the ghost red cell mediated method (Furusawa, M.,et.al., 1976, Methods. Cell. Biol.,14: 73-80; Straus, S. and Raskas, H., 1980, J. Gen. Virol. 48: 241-245; Godfrey, W., et.al., 1983, Proc. Natl. Acad. Sci. U.S.A. 80: 2267-2271 ), the bacterial protoplast fusion method (Chu, G. J. and Sharp, P. A., 1981, Gene 13:197-202; Sandri-Goldin, R. M., et.al., 1981, Mol. Cell. Biol. 1:743-752; Oi, V. T., and Morrison, S. L., 1986, Biotechniques 4: 214-221), the reconstituted Sendai virus envelope method (Loyter, A., et. al., 1984, Ciba. Found. Symp., 103:163-180), the laser-beamporation (Tsuka koshi, M., et.al., 1984, Appl. Phys. B., 35: 2284-2289; Tao, W., et.al., 1987, Proc. Natl. Acad. Sci. U.S.A. 84: 4180-4184), the electroporation method (Neumann, E., et. al., 1982; EMBO. J., 1 :841-845; Potter, H., et.al., 1984, Proc. Natl. Acad. Sci. U.S.A. 81: 7161-7165), the tungsten microprojectile method (Klein, T. M., et. al., 1987, Nature 327: 70-73), the retrovirus vector method (Jaenisch, R., 1976, Proc. Natl. Acad. Sci. U.S.A., 73: 1260-1264: Jahner, D. and Jaenish, R., 1980, Nature 287:456-458).

According to the preferred embodiment of the invention, that is the TFT cassette integration in the nucleic acid sequence of a living cell, the use of techniques such as homologous recombination or site-specific recombination are preferred. Such techniques are well known in the art. According to the invention the technique described by Sorrell DA and Kolb AF., ("Targeted modification of mammalian genomes" Biotechnol Adv. 2005 Nov; 23(7-8):431-69) is preferred.

According to the first step of the invention, living cells can be grown according to any known culture method as long as the method is appropriate to the used living cells. One skilled in the art will have no difficulty to find the good culture conditions appropriate to the cells he is studying.

According to the second step of the method of the invention, said cells obtained in step 1 are collected and mixed with said TFO probes in conditions that permit the formation of nucleic acid triplex.

According to the method of the invention the TFT sequences contained in the TFT cassette when inserted in the nucleic acid sequence of a living cell will have to be recognized by a molecular probe made of a specific sequence complementary to said TFT sequence, said specific complementary sequence being referred as Triplex Forming Oligonucleotide (TFO) and will have to form a stable complex in the form of a triple helix with said TFO.

According to the invention said TFO can be included in a construction referred to as the Triplex Forming Oligonucleotide probe (TFO probe). Said TFO probe can comprise two interactions poles: at one end, a so-called specificity head comprising a sequence able to form a specific triple helix with a complementary TFT sequence (the TFO) and at the other end, one (or several) capture handle(s), preferably more than one, advantageously 2, that can form a tight complex with a cognate capture hook eventually fixed on a support, for example at the surface of magnetic beads. A long spacer can connect the two interactions poles of the TFO probe.

According to a preferred embodiment of the invention one TFO probe can comprise one TFO. This implies that according to the invention several distinct TFO probes can be used. The number of distinct TFO probes that can be used is up to the number of different TFT sequences present in the TFT cassette.

According to the invention when the TFT sequences in the TFT cassette are a unique repeated sequence then the used TFO in TFO probe can be a unique sequence complementary to the TFT sequence.

According to the invention when in the TFT cassette the TFT sequences are multiple different sequences then the used TFOs in the TFO probes can be multiple different sequences each complementary to each different TFT sequences.

Many studies have been performed on the specificity of TFT sequences. According to these studies, structural requirements influence the design of TFOs and have led to the classification in different subtypes with individual binding properties of the specificity head of the TFOs that is the specific molecular probe that recognize the TFT sequences.

Triplex-forming oligonucleotides (TFOs) bind in the major groove of polyPy-polyPu sequences allowing specific targeting of the double-stranded DNA.

It is known that most of the time DNA exists as a duplex formed by anti-parallel chains hold together via the Watson-Crick base pairing scheme: A/T, G/C.

But it is also known that DNA duplexes containing Poly-Purine (poly-Pu) tracks in one strand and poly-Pyrimidine (poly-Py) tracks in the complementary strand, or the inverse, are able to form triplexes with either poly-Py or poly-Pu oligonucleotides by means of Hoogsteen base pairs.

Thus according to the invention, regardless of the nature of the TFT sequences (double strand poly-Pu/poly-Py sequences) contained in the TFT cassette they can be recognized via Hoogsteen base pairs by a poly-pyrimidine TFO and / or by a poly-purine TFO.

Another aspect of the invention is a method to improve the purification of Col.

One of the advantages of the invention is that it is possible to use more than one, at least 2 different, TFT sequences among the TFT sequences present in the TFT cassette. This involves that it will be possible to use more than one TFO probe, at least two different TFO probes. This suggests that the realization of the second step of the method of the invention can be performed in two different ways.
- a first way wherein in the second step cells obtained in step 1 are collected and mixed simultaneously with all molecular probes (the TFO probes) specific of the introduced TFT sequences of the TFT cassette introduced at the first step in conditions that permit the formation of nucleic acid triplex and the third step of the method immediately performed after the formation of nucleic acid triplex;
- a second way wherein the method is performed in as many cycle of formation of nucleic acid triplex/isolation/elution of the Col, as there are different TFO probes. For example in a first stage of the second step cells obtained in step 1 are collected and first mixed with one of the molecular probes (first TFO probes) specific of one of the introduced TFT sequences of the TFT cassette introduced at the first step in conditions that permit the formation of nucleic acid triplex, immediately followed by a second stage wherein the nucleic acid triplex formed in the first stage of the second step are isolated and Col eluted. Then said isolated Col are mixed with a second molecular probes (second TFO probes) specific of a second introduced TFT sequences of the TFT cassette introduced at the first step in conditions that permit the formation of nucleic acid triplex, immediately followed by a step wherein said nucleic acid triplex formed in the second stage of the second step are isolated and Col eluted. It appears obvious to those skilled in the art that in this variant of the second step of the method of the invention, the steps of mixing a new nucleic acid probe with a nucleic acid triplex previously formed in a previous stage can be repeated as many times as desired, especially as many times as there are different probes. Proteins bound to the finally isolated nucleic acid triplex can be then analyzed.

According to the second step of the invention cells can be collected according to any known method such as for example methods as described in Molecular cloning: a laboratory manual; (Joseph Sambrook, David William Russell, Cold Spring Harbor Press, Cold Spring Harbor, New York, 2001). Preferably cells are collected by scraping and centrifugation.

At this step as the collected cells generally form a more or less compact cluster, it may be worthwhile to add a fragmentation step before adding the TFO probe. In this way many mechanical (shearing methods), enzymatic or sonic techniques can be used. Sonication methods are preferred according to the invention.

This fragmentation step can improve the following step of triplex formation between the TFO probes and the TFT sequences.

According to the second step of the invention, once collected, eventually fragmented, cells have to be put in contact with TFO probes in a way that facilitates the recognition of the TFT sequences by the TFOs. Any method that leads to the triplex formation between the TFO probe and the TFT sequence can be used according to step 2 of the method of the invention.

In one preferred embodiment of this second step of the invention the recognition of the TFT sequences by the TFOs can be facilitated in purifying or isolating the cells nuclei prior to mixing it with the TFO. By purifying or isolating the nuclei it should be understood that said collected cells are subjected to treatment such as their nucleic acids and proteins or eukaryotic cell nuclei are at least partially isolated from their cellular environment. The skilled person knows many techniques to achieve such a result. Such techniques are well described in the literature of biology and / or molecular biology.

As previously said the TFO probe can contain two interactions poles: at one end, a so-called specificity head (including the TFO), then a long spacer, and at the other end, one (or several) capture handle(s) that can form a tight complex with a cognate capture hook.

According to the invention, the TFO in the TFO probe can be any nucleic acid sequence, complementary to at least one of the inserted TFT sequence, alone or combined with other elements that increase its effectiveness.

It has been reported that stabilization of triplex formation and high recognition specificity of a selected target site (Triplex Forming Tag site) can be achieved by the following modifications of the TFO probe:
1- Introduction of locked nucleic acids (LNA) nucleotides mixed with normal nucleotides in the TFO: Locked Nucleic Acids (LNA) comprises ribonucleotides with a 2'-0,4'-C-methylene linkage. LNA-containing TFOs have been recently described to effect very significant triplex stabilization. More precisely, previous works have thus shown that alternating LNA and normal nucleotides in TFO sequences is appropriate for triplex formation. Thus according to the invention, the TFO can comprise preferably nucleic acids (LNA) nucleotides mixed with normal nucleotides (Alexei A. et al., (1998). "LNA (Locked Nucleic Acids): Synthesis of the adenine, cytosine, guanine, 5-methylcytosine, thymine and uracil bicyclonucleoside monomers, oligomerisation, and unprecedented nucleic acid recognition". Tetrahedron 54 (14): 3607-30; Satoshi Obika et al. (1998). "Stability and structural features of the duplexes containing nucleoside analogues with a cross-linked N-type conformation, 2'-O,4'-C-methyleneribonucleosides". Tetrahedron Lett. 39 (30): 5401-4).
2- Replacement of cytosine by 5-methyl cytosine in the TFO.
3- Addition of a chemical compound composed by any aromatic ring structure working as an intercalator at one of the end (3' or 5') of TFO.

Thus according to one preferred embodiment of the invention, the TFO in the TFO probe can be a nucleic acid sequences comprising locked nucleic acid (LNA) nucleotides mixed with normal nucleotides.

In another preferred embodiment of the invention, cytosines can be replaced by 5-methyl cytosine in the TFO.

In a very preferred embodiment of the invention the TFO in the TFO probe can be a nucleic acid sequences comprising locked nucleic acid (LNA) nucleotides mixed with normal nucleotides and cytosines can be replaced by 5-methyl cytosine in the TFO.

In one of the very preferred embodiment of the invention the TFO can be a nucleic acid sequences comprising locked nucleic acids (LNA) nucleotides mixed with normal nucleotides, that can comprise at one of its end (3' or 5') an aromatic ring structure, for example an intercalating agent and in which cytosines can be replaced by 5-methyl cytosines.

According to the invention, the so-called specificity head can be made of an intercalator linked directly or through a short spacer to the TFO. Said intercalator can be a chemical compound composed by any aromatic ring structure, for example an intercalating agent such as psoralen, acridine, ethidium bromide, berberine, proflavine, daunomycin, doxorubicin, thalidomide, quinacrine, or orthophenanthroline, preferably psoralen. Preferably said chemical compound can be a photo-activable compound, most preferably a photo-activable intercalating agent. This will permit, once the chemical compound is intercalated into the double stranded TFT nucleic acid, to covalently link said compound to the nucleic acid, using a photon emissive source. This will reinforce the strength of the TFO/TFT complex thus facilitating the recovery of the Protein/nucleic acid complex in the final purification step.

According to the invention, the so-called long spacer that joined the so-called specificity head to the capture handle can be of any type of known spacers, preferably a carbon spacer that can have a length comprised between 1 to 300 carbon atoms, preferably between 100 to 200, most preferably between 110 to 130 carbon atoms.

According to the invention, the so-called capture handle can be a compound able to form a couple of strongly interacting molecules with another compound used as a hook. For example such a couple can be any kind of materials showing affinity interaction such as, combination of histidine-metal, antigen-antibody (e.g., FLAG-anti FLAG), specific oligonucleotide-specific oligonucleotide binding protein (e.g., IacO-LacI), and so on.

In a particular embodiment the capture handle can be a compound that can bind to another one used as a hook. Preferred hook can be streptavidin or an equivalent such as Avidin or Neutravidin and preferred capture handle can be biotin or equivalent such as desthiobiotin.

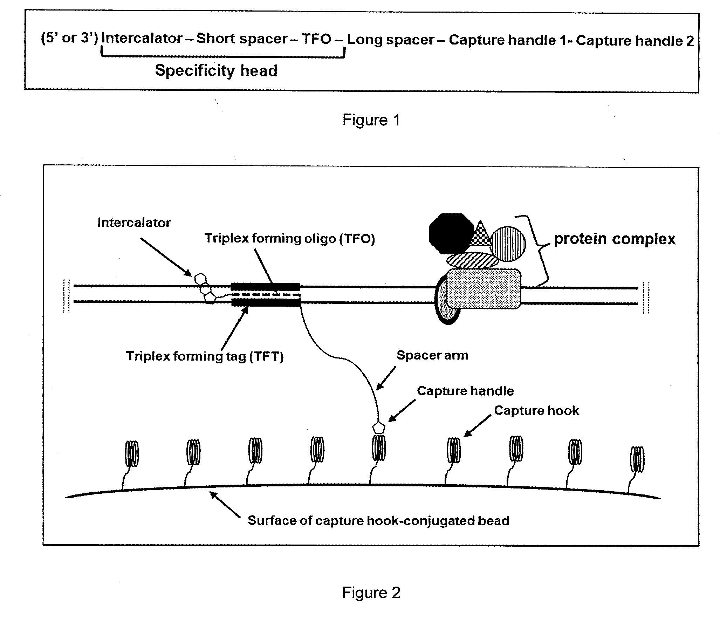

In a very preferred embodiment of the invention (as shown in figure 1), the TFO probe can contain at one of its ends the so-called specificity head including an intercalator linked directly or through a short spacer to the TFO. Said so-called specificity head can be directly linked by a long spacer to several different, at least 2 different, capture handle(s).

Using different capture handles in the TFO probes allows using different protocols (sequential or simultaneous capture) to capture the probes (capture protocol) and particularly sequential capture protocol to improve the recovery yield of the chromatin fragment of interest and to improve the signal over noise ratio.

Different capture protocols will be now described based on the use of TFT cassettes including only 2 different TFT sequences. It is evident that these protocols can be easily adapted and used with TFT cassettes comprising more than 2 TFT sequences. Particularly the number of TFO probes can be easily adapted to the number of different TFT sequences present in the TFT cassette. In the same way the number of steps of the protocol can be easily adapted.

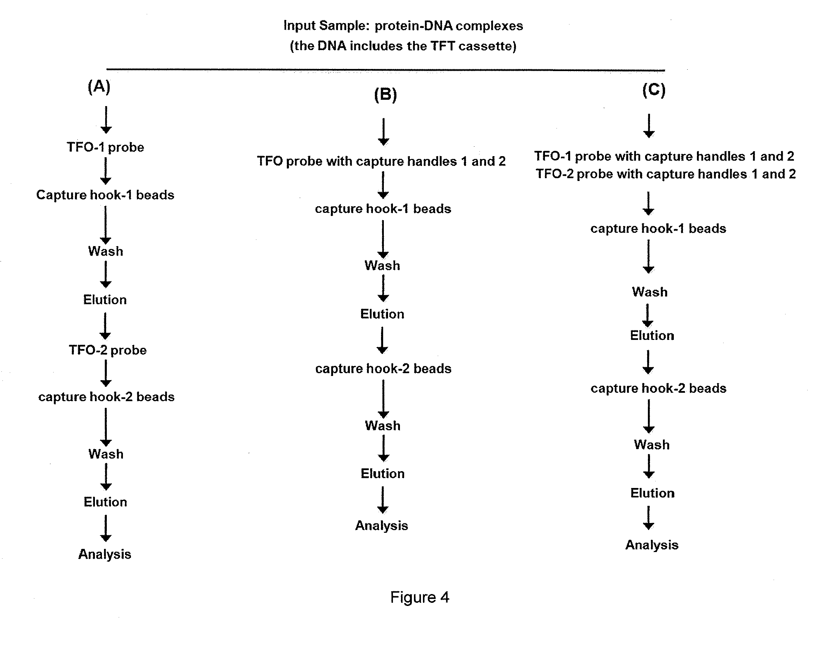

As example a first embodiment of the capture protocol (Figure 4A) can consist in using successively two different TFO probes comprising two different TFO (TFO-1 probe and TFO-2 probe) to capture a fragment of chromatin containing a TFT cassette that contains 2 different TFT sequences (TFT-1 and TFT-2 sequences). Each TFO probe also comprises two different capture handles (CH-1 and CH-2) each specific of two different capture hooks (capture hook-1 and capture hook-2) showed in this example that is not limitative linked to magnetic beads. Cells obtained in the first step of the method according to the invention, are first incubated with the TFO probe-1 and then incubated with capture hook-1. Captured fragment of chromatin of interest can be then washed and bound fragment of chromatin of interest can be eluted from the magnetic beads. Then said eluted fragment of chromatin of interest can be further purified by a similar cycle involving TFO probe-2 and capture hook-2, and then fragment of chromatin of interest can be washed, eluted from the magnetic beads and analyzed.

As example a second embodiment of the capture protocol (Figure 4B) can consist in using a single TFO-probe that carries two distinct capture handles (CH-1 and CH-2). Said single TFO-probe comprises a TFO sequence that is specific of a the TFT sequence repeated at least twice in the TFT cassette inserted in the cell DNA at step 1 of the method of the invention. Cells obtained in the first step of the method according to the invention, can be incubated with said TFO-probe and then incubated with capture hook-1 (that binds to CH-1) showed in this example that is not limitative linked to magnetic beads Captured fragment of chromatin of interest can be washed and bound fragment of chromatin of interest can be eluted from the magnetic beads. Following elution, said fragment of chromatin of interest can be further incubated with capture hook-2 (that binds to CH-2) and then fragment of chromatin of interest can be washed, eluted from the magnetic beads and analyzed.

As example a third embodiment of the capture protocol (Figure 4C) can consist in a combination of the above first and second embodiment of the capture protocol. In this third example of capture protocol cells obtained in the first step of the method according to the invention, are simultaneously incubated with two different TFO probes (TFO probe-1 and TFO probe-2) that both contain two distinct capture handles (CH-1 and CH-2). After incubation a first capture step is performed with capture hook-1 (that binds to CH1) showed in this example that is not limitative linked to magnetic beads. Then the isolated fragment of chromatin of interest can be washed and bound fragment of chromatin of interest can be eluted from the magnetic beads. Following elution, said fragment of chromatin of interest can be further incubated with capture hook-2 (that binds to CH2) showed in this example that is not limitative linked to magnetic beads and then fragment of chromatin of interest can be washed, eluted from the magnetic beads and analyzed.

According to the invention, in the second step of the method, a nucleotide Triplex structure has to be formed. Any conditions that permit such a formation can be used according to the invention. One of the preferred methods that can be used according to the invention is the one described in Brunet et al. (Nucleic Acid Research, 2005, Vol. 33, N°13, 4223-4234).

According to the third step of the method of the invention, the nucleic acid triplex formed in second step can be isolated according to any known methods. Preferred method can be a method in which a hook, specific to the capture handle, is used to bind to the capture handle. Such methods can be for example combination of histidine-metal, antigen-antibody (e.g., FLAG-anti FLAG), specific oligonucleotide being able to form a double-helix with an oligonucleotide of complementary sequence, specific oligonucleotide-specific oligonucleotide binding protein (e.g., IacO-LacI) and so on.

According to one of the preferred embodiment of the invention where the capture handle is a biotin or an equivalent, the hook can be a streptavidin.

To facilitate the purification of the captured triplex the hook can be fixed on a column or on beads, for example magnetic beads. The use of the magnetic beads is the preferred purification method used according to the invention. Such methods are described in many references such as Déjardin and Kingston (Cell 136, 175-186, January 9, 2009).

According to a specific alternative form of the invention, a cross-linking step of the proteins bound to the nucleic acid can be added to the method. This cross-linking step can be added just after step 1 that is before the collecting step or can be added just after the collection of the cells that is just before adding the TFO probe.

This step can be of importance because during cross-linking the proteins surrounding the nucleic acid are cross-linked between-them and between them and said nucleic acid.

According to the invention, any method known in the art that permits the protein-protein and/or protein-nucleic acid crosslink can be used as for example for Protein-DNA crosslink: UV photo crosslinking, formaldehyde cross-linking technique, Hexavalent chromium and for Protein-Protein for further analysis by combined with Protein-DNA crosslink: dimethyl adipimidate (DMA); disuccinimidyl suberate (D88); dithiobis[succinimidyl propionate] (D8P); ethylene glycolbis[succinimidyl succinate] (EG8).

Preferably the *in vivo* formaldehyde cross-linking technique can be used according to the method described by Orlando V et al. (Methods. 1997 Feb; 11 (2):205-14).

It is noteworthy that the cross-linking step can be not necessary, if the interactions between proteins or between proteins and nucleic acids are strong enough so that it is not necessary to conduct an additional cross-linking step.

According to another specific alternative form of the invention, to optimize the method a cell lysis step can be added to the method. According to which embodiment of the invention is chosen (with or without cross-linking step), this cell lysis step can be added at different times in the method of the invention.

When no cross-linking step is performed according to the method, this cell lysis step can be added once the cells have been collected in the second step of the method.

When a cross-linking step is performed according to the method, this cell lysis step can be added indifferently before or after the cross-linking step, preferably before.

Notwithstanding the embodiment of the method used (with or without cross-linking step), the cell lysis step can be performed as according any known manner, preferably according to the method described in the following references.,("Association of RNA polymerase with transcribed regions in Escherichia coli", Wade JT, and Struhl K.; Proc Natl Acad Sci USA. 2004 Dec 21;101(51):17777-82 or "Cockayne syndrome A and B proteins differentially regulate recruitment of chromatin remodeling and repair factors to stalled RNA polymerase II in vivo", Mol Cell. 2006 Aug; 23(4):471-82).

The invention also relates to the use of the method of the invention for the preparation of nucleotide-protein complex.

The invention finally relates to a kit for the implementation of the method of the invention, said kit comprising at least one TFT Cassette to be introduced near the Col in the nucleic acid sequence of a living cell said TFT cassette being as described previously in the present text, at least one cross-linking compound, at least one molecular probe (the TFO probes) specific of at least one of the TFT sequences contained in the TFT cassette, said TFO probes being as described previously in the present text, and at least a hook constituted by a compound that can bind to the TFO's capture handle. If the TFO probes comprise more than one capture handles, the kit can comprise as many hooks.

The present invention will be better understood, and its details more clearly apparent, on reading the following examples and description in relation to the figures in which:
Figure 1 shows an example of one possible TFO probe: The left end of the TFO probe contains the specificity head which is formed by an intercalator followed by a short spacer grafted to the TFO which is an oligonucleotide. The right end of the TFO probe is made of two contiguous capture handles (1 and 2). The specificity head and the capture handles are linked by a spacer composed of a linear chain of carbon atoms.
Figure 2 is a schematic representation of the overall approach of the method according to the invention. The upper part of the figure shows the Chromatin-of-Interest fragment (Col fragment) with the protein complex to be analyzed bound to the double strand DNA. To simplify the figure only one TFT sequence (TFT tag : ■) of a non-represented TFT cassette is shown, inserted in the DNA. The stable complex in the form of a triple helix is shown with the specific oligonucleotide probe TFO (- - - - -) intercalated between the 2 strands of the TFT sequence. Also shown are the intercalator grafted on one end of the TFO and the long spacer arm grafted on the opposite end of the TFO. At the opposite end of the long spacer arm the capture handle is shown bound to the corresponding capture hook represented fixed on the surface of a bead.

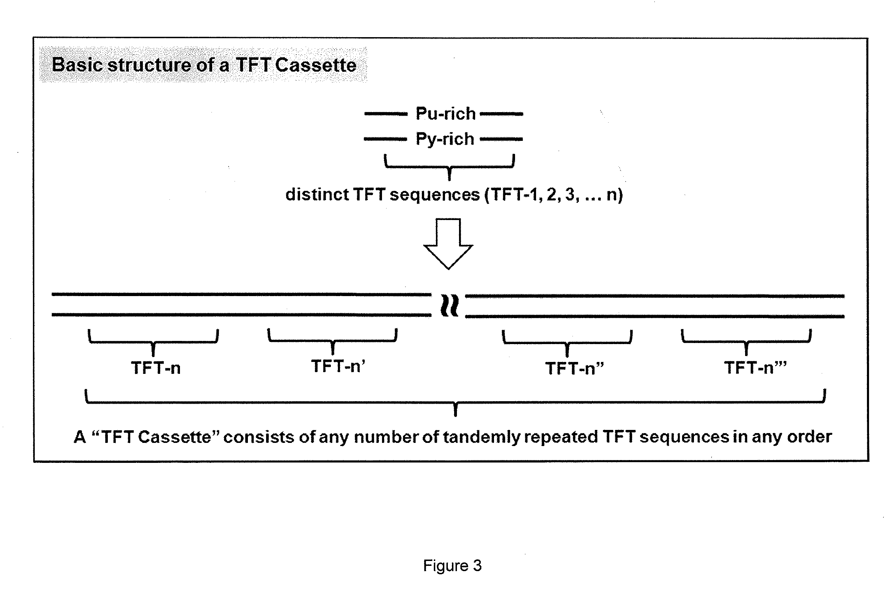

Figure 3 shows a schematic representation of the structure of a TFT cassette showing that according to the invention the TFT cassette consists of any number of repeated TFT sequences in any order.
Figure 4 shows a schematic diagram of the capture protocols described in the text exemplifying the successive use of 2 different TFO probes (4A), the use of one TFO probe with 2 different capture handles (1 and 2) and successive step of capture first with a capture hook-1 corresponding to the capture handle 1 and second with a capture hook-2 corresponding to the capture handle 2 (4B) and the simultaneous use of 2 different TFO probes(TFO-1 and TFO-2) each with 2 different capture handles (1 and 2) and successive step of capture first with a capture hook-1 corresponding to the capture handle 1 and second with a capture hook-2 corresponding to the capture handle 2 (4C).

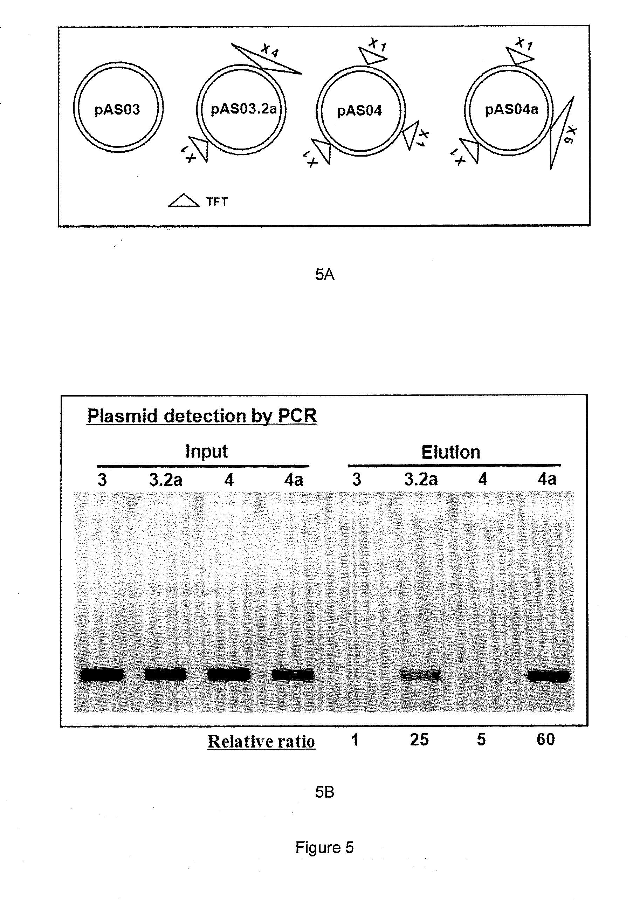

Figure 5 shows the results obtained in the assay described in example 1.
Figure 5A shows plasmid constructions used in the assay.
   - The control pAS03 plasmid, that contains no TFT cassette, is derived from pcDNA3.1 (+)CAT (Invitrogen).
   - Plasmid pAS03.2a is derived from pAS03 by inserting TFT sequences/cassettes at two locations. One site (x1) contains a single TFT sequence and the other contains one TFT cassette containing 4 TFT sequence (x4).
   - Plasmid pAS04 is derived from pAS03 by inserting 3 single TFT sequences (x1) at three distinct locations.
   - Plasmid pAS04a is derived from pAS03 by inserting, at 3 distinct locations, two single TFT sequence (x1) and one TFT cassette containing 6 TFT sequence (x6).
Figure 5B shows the results obtained in example 1 for the *in vivo* capture of plasmids containing TFT sequence/cassettes and showing the effect of using TFT cassettes versus TFT sequence (agarose gel electrophoresis stained with ethydium bromide). Each "input lane" corresponds to the PCR amplification of 0.1 ng of total DNA in the input sample. Each "elution lane" corresponds to the PCR amplification of 0.1 % of eluted sample.
Figure 6: shows the results obtained in the assay described in example 2.

The upper part of the figure shows plasmid constructions used in the assay.
- The control pAS03 plasmid, that contains no TFT cassette, is derived from pcDNA3.1 (+)CAT (Invitrogen).
- Plasmid pAS04a is derived from pAS03 by inserting, at 3 distinct locations, 2 single TFT sequence (x1) and one TFT cassette containing 6 TFT sequence (x6).

The lower part of the figure shows the results obtained in example 2 for the in vivo capture of plasmids containing TFT sequence/cassettes and showing the effect of multiple TFO probes (agarose gel electrophoresis stained with ethydium bromide).

Each "input lane" corresponds to the PCR amplification of 0.1 ng of total DNA in the input sample. Each "elution lane" corresponds to the PCR amplification of 0.05 % of eluted sample.

### Example 1: in vivo capture of plasmids containing TFT-sequence/cassettes and effect of using TFT cassette versus TFT sequence.

This experiment aims at investigating the effect of using TFT cassette versus TFT sequence on the efficiency of recovery of the target plasmid transfected into human cells.

Human cells, 293FT (Invitrogen), are chemically transfected with different TFT-sequence/cassettes containing plasmids:
- The control pAS03 plasmid, that contains no TFT sequence/cassette, is derived from pcDNA3.1 (+)CAT (Invitrogen).
- Plasmid pAS03.2a is derived from pAS03 by inserting TFT sequence/cassettes at two locations. One site contains a single TFT sequence (x1) and the other contains one TFT cassette containing 4 TFT sequence (x4).
- Plasmid pAS04 is derived from pAS03 by inserting 3 single TFT sequences (x1) at three distinct locations.
- Plasmid pAS04a is derived from pAS03 by inserting, at 3 distinct locations, two single TFT sequence (x1) and one TFT cassette containing 6 TFT sequence (x6).

The human cells are cultivated under a normal growth conditions and cross-linked with HCHO at the time of harvest. The cross-linked cells are treated with buffer containing mild detergents to destroy the cellular membrane. The nuclei are isolated by means of centrifugation and disrupted by sonication. The soluble fraction (supernatant) is recovered from the mixture by centrifugation. The supernatant is first incubated with streptavidin beads to remove intrinsically-biotinylated human proteins, and the resulting supernatant is referred to as the input sample. The input (from 6.5 x 10⁵ cells equivalent) is incubated with 2 different TFO-probes (TFO-1 and TFO-2) and subsequently mixed with streptavidin beads.

The beads are washed and DNA/protein complexes are eluted from the beads under mild conditions. The eluted products are reverse cross-linked by heat treatment. Plasmid recovery efficiency is assessed by PCR amplification (semi-quantitative assay) of a specific DNA fragment that belongs to the target plasmid. PCR products are analyzed following agarose gel electrophoresis by ethydium bromide staining.

In figure 5B, each "input lane" corresponds to the PCR amplification of 0.1 ng of total DNA in the input sample, and each "elution lane" corresponds to the PCR amplification of 0.1 % of eluted sample.

The results show that plasmid capture is highly specific for TFT containing plasmids as no visible band is seen for the control plasmid pAS03 (elution, lane 3).

There is a distinct increase in the capture efficiency as a function of the number of TFT sequences inserted in the plasmids.

It also appears that the use of TFT cassette (3.2a and 4a) strongly increase the capture efficiency.

This suggests that the presence of TFT cassette increase the chances at least one TFT sequence be located in a nucleosome linker region thus favoring triple helix formation.

The relative amounts of plasmid captured in the elution lanes illustrate the benefit of using TFT cassettes. For plasmid pAS04a, the absolute recovery efficiency corresponds to about 6% of input plasmid.

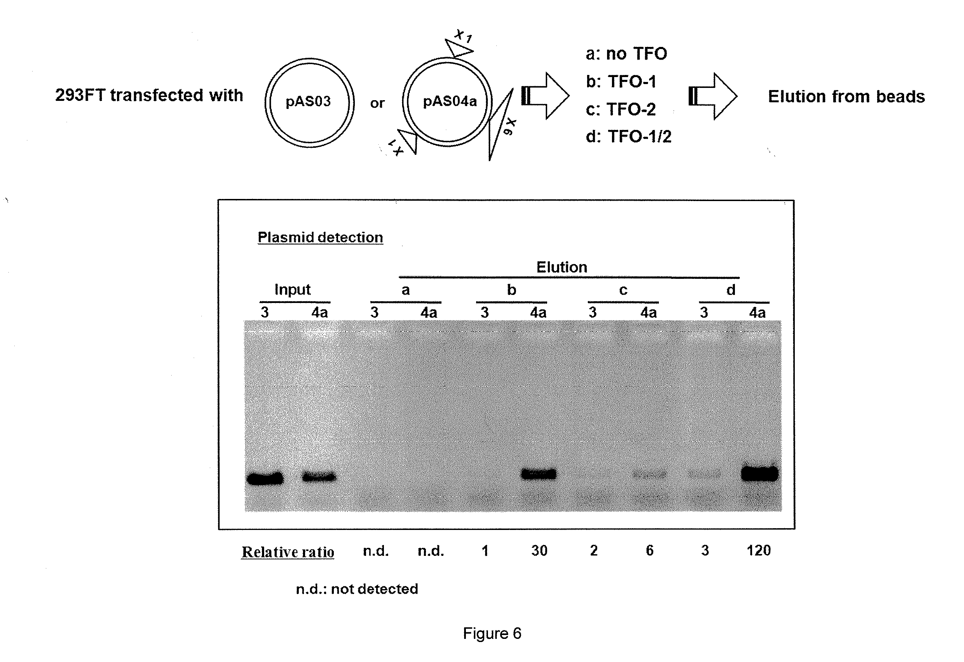

### Example 2: In vivo capture of plasmids containing TFT-sequence/cassettes and effect of using multiple TFO probes (Fig. 6).

This experiment aims at investigating the beneficial effect of using two different TFO probes as compared to a single TFO probe on the efficiency of recovery of target plasmid transfected into human cells.

The experimental procedures (summarized in the upper part of figure 6) are same as in example 1 (fig. 5) except for the addition of distinct combinations of TFO probes:
- Condition a: no TFO probe was added;
- Condition b: only one type of TFO probe was added (TFO-1);
- Condition c: only one type of TFO probe was added (TFO-2);
- Condition d: a mixture of the two types of probes was added (TFO-1 and TFO-2).

With respect to plasmid DNA analysis by semi-quantitative PCR, each lane corresponds to the PCR amplification the same fraction of eluted sample (0.05%). Efficiency of elution strongly depends on both the use of TFT cassette (instead of TFT sequences) as well as on the simultaneous use of 2 different TFO-probes (TFO-1 and TFO-2).

In the lower part of figure 6, each lane corresponds to about 1 X 10⁴ cells. PCR amplifications were performed in input samples on 50 pg of total DNA as a template. In elution samples 0.05% of elution products are used as a template.

In case of pAS04a in the condition d, about 12% of plasmid included in the input are recovered in the elution sample.

The use of multiple TFO probes strongly increases plasmid capture efficiency. The efficiency of TFO-2 alone (elution c) appears to be poor while its use in combination with TFO-1 synergistically improves the overall capture efficiency (elution d compared to b). In vitro experiments have shown that TFO-1 and TFO-2 exhibit similar triplex formation capacities (data not shown).

## Claims

1. Method for the isolation of the proteins bound to any kind of interesting nucleic acid sequence (Chromatin of Interest (Col)), wherein
- in a first step a Triplex Forming Tag cassette (TFT cassette) is introduced in said nucleic acid sequence of a living cell and said living cells are grown;
- in a second step cells obtained in step 1 are collected and mixed with at least one molecular probe (the TFO probe) specific of at least one of the introduced TFT sequences of the TFT cassette in conditions that permit the formation of nucleic acid triplex;
- in a third step the nucleic acid triplex formed in second step are isolated and bound proteins are analyzed,
**characterized in that** the TFT cassette comprises at least 2 TFT sequences.

2. The method according to claim 1, wherein said TFT cassette comprises at least 2 TFT sequences, advantageously between 2 and 12 preferably between 3 and 10, more preferably between 5 and 7 TFT sequences.

3. The method according to any one of claims 1 or 2, wherein said TFT sequences is under the form of a single double strand Poly-Purine (poly-Pu)/poly-Pyrimidine (poly-Py) or Poly- Pyrimidine (poly-Py) /poly-Purine (poly-Pu) sequence.

4. The method according to any one of claims 1 to 3, wherein in said TFT cassette the TFT sequences are all identical or all different or a mixture of identical and different TFT sequences.

5. The method according to any one of claims 1 to 4, wherein said TFT sequence has a length comprised between 10 to 50 base pairs, preferably between 15 to 35 base pairs very preferably of about 20 base pairs long.

6. The method according to any one of claims 1 to 5, wherein in the TFT cassette said TFT sequences are contiguous or separated by a spacer made of 1 to 20, preferably between 1 to 10 nucleotide base pairs.

7. The method according to any one of claims 1 to 6, wherein the TFO probe comprises at one end a specificity head comprising at least a sequence able to form a specific triple helix with a complementary TFT sequence (the Triplex Forming Oligonucleotide: TFO), a spacer and at the other end, one or several capture handle(s), advantageously 2 capture handles.

8. The method according to claim 7, wherein said capture handle is a compound able to form a couple of strongly interacting molecules with another compound used as a hook.

9. The method according to claim 8 wherein said couple is a combination of histidine-metal, antigen-antibody (e.g., FLAG-anti FLAG), specific oligonucleotide being able to form a double-helix with an oligonucleotide of complementary sequence, specific oligonucleotide-specific oligonucleotide binding protein (e.g., IacO-LacI), or a compound that can bind to another one used as a hook such as biotin or equivalent such as desthiobiotin and Avidin or Neutravidin.

10. The method according to claim 7, wherein the specificity head further comprises an intercalator linked directly or through a short spacer to the TFO.

11. The method according to claim 7, wherein said TFO is a nucleic acids sequence comprising locked nucleic acid (LNA) nucleotides mixed with normal DNA nucleotides.

12. The method according to any one of claims 7 or 11, wherein in said TFO cytosines are replaced by 5-methyl cytosine.

13. The method according to any one of claims 1 to 12, wherein in step 2 a mixture of at least 2 different TFO probes is used.

14. Use of the method according to anyone of the claim 1 to 13 for the preparation of nucleotide-protein complex.

15. A kit for the implementation of the method according to anyone of the claim 1 to 14, said kit comprising at least one TFT cassette to be introduced near the Col in the nucleic acid sequence of a living cell, at least one cross-linking compound, at least one molecular probe specific of the TFT cassette, and at least a hook constituted by a compound that can bind to the TFO's capture handle, said TFT cassette, said cross-linking compound, said molecular probe specific of the TFT sequence and said hook being as described in any one of claims 1 to 13.
